# FASCICULE DE BREVET EUROPEEN

(11) **EP 2 449 377 B1**
(45) Date de publication et mention de la délivrance du brevet: **21.06.2017**
(21) Numéro de dépôt: 10729854.9
(22) Date de dépôt: 30.06.2010
(51) Int. Cl.: G01N 1/30, G01N 33/574

(54) **MÉTHODE DE DÉTECTION DE CELLULES TUMORALES PAR SIGNAL DE FLUORESCENCE**
VERFAHREN FÜR DEN NACHWEIS VON TUMORZELLEN ANHAND VON FLUORESZENZSIGNALEN
METHOD OF DETECTING TUMOUR CELLS BY FLUORESCENCE SIGNALS

(30) Priorité: 30.06.2009 EP 09164258
(43) Date de publication de la demande: 09.05.2012
(73) Titulaire: Assistance Publique - Hôpitaux de Paris, 75004 Paris (FR); Centre National de la Recherche Scientifique, 75016 Paris (FR); Université Paris-Sud 11, 91405 Orsay Cedex (FR)
(72) Inventeur: FABRE, Monique, F-75013 Paris (FR); FERLICOT, Sophie, F-75014 Paris (FR); FONTAINE AUPART, Marie-Pierre, F-94260 Fresnes (FR); STEENKESTE, Karine, F-91140 Villebon sur Yvette (FR); ESCHWEGE, Pascal, F-94240 l'Hay les Roses (FR)
(74) Mandataire: Le Guen-Maillet
(86) Numéro de dépôt international: PCT/EP2010/059324
(87) Numéro de publication internationale: WO 2011/000894

(56) Documents cités:
- TROPE C ET AL: "Colony-forming ability of human ovarian carcinomas in the courtenay soft agar assay: Relationship to clinical parameters, histopathology, and DNA pattern" GYNECOLOGIC ONCOLOGY, ACADEMIC PRESS, LONDON, GB LNKD- DOI:10.1016/0090-8258(89)91006-8, vol. 32, no. 1, 1 janvier 1989 (1989-01-01), page 128, XP022990320 ISSN: 0090-8258 [extrait le 1989-01-01]
- TANG G C ET AL: "Spectroscopic differences between human cancer and normal lung and breast tissues." LASERS IN SURGERY AND MEDICINE 1989 LNKD- PUBMED:2733538, vol. 9, no. 3, 1989, pages 290-295, XP002605430 ISSN: 0196-8092
- FRABLE W J ET AL: "Current practice of urinary bladder cytology." CANCER RESEARCH AUG 1977 LNKD- PUBMED:872107, vol. 37, no. 8 Pt 2, août 1977 (1977-08), pages 2800-2805, XP002605431 ISSN: 0008-5472
- HEMSTREET G P ET AL: "Intravesical CDDP therapy compared with combined CDDP and external radiation in noninvasive bladder cancer Monitored with quantitative fluorescence cytology" UROLOGY, BELLE MEAD, NJ, US LNKD- DOI:10.1016/0090-4295(84)90389-3, vol. 24, no. 1, 1 juillet 1984 (1984-07-01), pages 59-63, XP023282244 ISSN: 0090-4295 [extrait le 1984-07-01]
- Karine Steenkeste ET AL: "Ex Vivo Fluorescence Imaging of Normal and Malignant Urothelial Cells to Enhance Early Diagnosis", Photochemistry and Photobiology, vol. 83, no. 5, 1 September 2007 (2007-09-01), pages 1157-1166, XP055161947, ISSN: 0031-8655, DOI: 10.1111/j.1751-1097.2007.00079.x
- G. N. Papanicolaou: "A NEW PROCEDURE FOR STAINING VAGINAL SMEARS", Science, vol. 95, no. 2469, 24 April 1942 (1942-04-24), pages 438-439, XP055260745, US ISSN: 0036-8075, DOI: 10.1126/science.95.2469.438
- WHITE ET AL: "TIME WAITS FOR NO MAN: DECIDING WHEN TO FILE A PATENT APPLICATION IN EUROPE", NATURE BIOTECHNOLOGY, NATURE PUBLISHING GROUP, US, vol. 25, no. 6, 1 June 2007 (2007-06-01), pages 639-641, XP009085067, ISSN: 1087-0156, DOI: 10.1038/NBT0607-639

## Description

L'invention se rapporte à une méthode de détection de cellules en division ou ayant un potentiel de division et de réplication comme les cellules transformées dans un échantillon cytologique coloré à l'aide d'une coloration de Papanicolaou, par la détection d'un signal de fluorescence au niveau des membranes de ces cellules. Ces cellules présentent donc une signature de fluorescence différente de celle des cellules normales et différenciées, et qui est corrélée à leur capacité à se diviser et se répliquer.

L'étude cytologique des cellules après fixation sur lames et coloration permet de caractériser les cellules présentes dans les échantillons biologiques lors de l'observation au microscope, et de poser des diagnostics quant à la présence de cellules tumorales.

La cytologie a deux grands domaines d'application : cytologie à grande échelle dite de dépistage (exemple : frottis cervico-utérins) et cytologie spécialisée chez des patients sélectionnés (exemples : frottis urinaires, cytologies d'organes profonds, ....). Le recueil des cellules se fait par étalement direct sur lames du produit cellulaire biologique ou, plus récemment en recueillant le produit cellulaire « en milieu liquide » dans le but d'augmenter la sensibilité du diagnostic et de disposer d'une réserve de cellules utiles pour permettre des techniques complémentaires (immunocytochimie, biologie moléculaire). Les lames de cytologie sont colorées, en routine, par la coloration de Papanicolaou.

La cytologie est une méthode particulièrement intéressante en clinique. Elle est en effet peu coûteuse et informative, car permettant de caractériser directement les cellules des organes potentiellement pathologiques ; elle est toutefois limitée par le nombre décroissant de cytotechniciens et de cytopathologistes formés et par le long apprentissage nécessaire à la maitrise de cette méthode diagnostique. En effet, la mise en oeuvre de la cytologie demandant des ressources humaines importantes est souvent remplacée par des méthodes de diagnostic histopathologiques en paraffine qui sont plus invasives pour le patient et plus coûteuses pour notre système de santé. De nouvelles techniques semi-automatisées d'aide à la lecture des frottis cervico-utérins se sont développées ces dernières années dans le domaine du dépistage du cancer du col.

Il serait donc particulièrement intéressant de disposer d'un procédé simple et performant, mettant en évidence un phénotype large et robuste des cellules transformées, en associant une observation en fluorescence à une coloration cytologique par ailleurs classique et largement appréciée en fond clair par les cytotechniciens et les cytopathologistes. La force de cette invention est d'apporter une nouvelle puissance diagnostique sans ajouter l'étape d'un marquage supplémentaire chronophage et onéreux. Ce procédé peut être en partie automatisable permettant une aide à la lecture pour que l'intervention humaine soit réduite uniquement à la vérification de zones de lames préalablement identifiées comme pouvant présenter des cellules pathologiques.

La cytologie est une méthode diagnostique sensible, principalement pour la détection des cancers de haut grade de malignité, mais apparaît par contre peu sensible pour le diagnostic des cancers de bas grade de malignité, correspondant à un groupe de lésions classées comme « modifications de nature indéterminée » dont le pourcentage varie en fonction des cellules analysées. Ces diagnostics incertains impliquent de répéter l'examen cytologique, donc de reconvoquer les patients avec les difficultés matérielles et morales que cela nécessite, ainsi qu'un risque de perdu de vue et une perte de chance dans certains territoires géographiques en raison d'une faible densité médicale. Pour pallier à ces difficultés, il serait utile de proposer un nouveau procédé diagnostique qui soit simple, reproductible, standardisé, peu coûteux et qui fasse gagner du temps au cytopathologiste.

Si on analyse la littérature sur les performances diagnostiques de la cytologie, on peut ainsi montrer les limites diagnostiques pour les lésions de bas grade de malignité.

Il est rappelé que :
Sensibilité : probabilité que le diagnostic soit positif chez les individus atteints par la maladie recherchée (détection des vrais positifs) : le test est positif si le patient est atteint. La sensibilité est faible quand le nombre de faux négatifs est élevé. La sensibilité se calcule par la formule SE = (nombre d'individus atteints chez lesquels le signe est présent) / (nombre d'individus atteints chez lesquels le signe est présent + nombre d'individus atteints chez lesquels le signe est absent).
Spécificité : probabilité que le diagnostic soit négatif chez les individus non atteints par la maladie recherchée (non-détection des vrais négatifs) : le test est négatif si le patient n'est pas atteint. La spécificité est faible quand le nombre de faux positifs est élevé. La spécificité se calcule par la formule SP = (nombre d'individus non-atteints chez lesquels le signe est absent) / (nombre d'individus non-atteints chez lesquels le signe est absent + nombre d'individus non-atteints chez lesquels le signe est présent).
Valeur prédictive positive : probabilité d'avoir la maladie si le test diagnostic est positif (c'est-à-dire que le patient ne soit pas un faux positif) : le patient est atteint si le test est positif. La valeur prédictive positive se calcule par la formule VPP = (nombre d'individus atteints chez lesquels le signe est présent) / (nombre d'individus atteints chez lesquels le signe est présent + nombre d'individus non-atteints chez lesquels le signe est présent).
Valeur prédictive négative : probabilité de ne pas porter la maladie si le test diagnostic est négatif (que le patient ne soit pas un faux-négatif) : le patient n'est pas atteint si le test est négatif. La valeur prédictive négative se calcule par la formule VPN = (nombre d'individus non-atteints chez lesquels le signe est absent) / (nombre d'individus non-atteints chez lesquels le signe est absent + nombre d'individus atteints chez lesquels le signe est absent).

La diminution du taux de lames apparaissant comme faussement négatives (augmentation de la valeur prédictive négative), ainsi que l'augmentation de la sensibilité (obtenue notamment par la diminution du nombre d'individus atteints chez lesquels le signe diagnostic est absent) sont deux éléments qui sont essentiels dans le développement d'un test cytologique remplissant les conditions mentionnées ci-dessus, et qui puisse ainsi être automatisable.

Dans un monde idéal, il convient en effet que le cytopathologiste puisse avoir accès (pour vérification et pose du diagnostic définitif) aux lames de tous les patients potentiellement atteints par la maladie recherchée (et que ces lames ne soient donc pas écartées, soit une valeur prédictive négative de 100 %), et que tous les patients atteints par la maladie répondent au diagnostic (sensibilité de 100 %). Ceci équivaut au fait qu'une non-réponse au diagnostic implique nécessairement que le patient n'est pas atteint.

En pathologie du col utérin, les lésions atypiques malpighiennes de nature indéterminée (ASC-US) ou ne pouvant exclure une lésion de haut-grade (ASC-H) selon le système Bethesda 2001, représentent respectivement de 2,3 à 5.4 % et 0.6 % des cas (Quddus MR, et al. Cytojournal. 2009 6;6:15. ; Amaral RG et al. Rev Bras Ginecol Obstet. 2008;30(11):556-60). Le taux de faux négatif est estimé à 6.88 % et celui des lésions atypiques de nature indéterminée de 10.78 % dans une série française (Labbé S, Petitjean A, Ann Pathol. 1999;19(5):457-62). Si on compare les données de la colposcopie et celles du frottis, la sensibilité globale de détection des CIN (néoplasies intraépithéliales) 2 et 3 est estimée à 68 % (Campion MJ et al. J Exp Clin Cancer Res 1990 (Suppl) : FC/107).

En pathologie mammaire, une ponction cytologique classée comme C3 (atypique ou de nature indéterminée) correspond à un risque de malignité d'environ 20% (NIH The uniform approach of breast fine needle aspiration biopsy. NIH consensus development Conférence. Am J Surg Pathol 1997;174:371-85).

En pathologie respiratoire, la sensibilité du brossage bronchique pour le diagnostic de cancer varie entre 57,8 et 71.6 % (Fan YB, Cytotechnology. 2010;62:53-9) selon la technique utilisée. La cytologie pleurale a une sensibilité de 35%, une spécificité de 100 % et une valeur prédictive négative de 82 %. (Davie HE et al. Am J Respir Crit Care Med. 2009;180(5):437-44.)

En pathologie thyroïdienne, la sensibilité de la cytologie pour le diagnostic de cancer est estimée entre 81 à 96 % selon la technique utilisée (Buley ID et al. Clin Oncol (R Coll Radiol). 2000;12(3):166-71 ; Cochand-Priollet B et al. Cytopathology 2003 Dec;14(6):343-9.

En pathologie bilio-pancréatique, les brossages de la voie biliaire ont une sensibilité faible de 18 à 67 % (Waugh MS, et al. Diagn Cytopathol. 2008;36(9):651-6). Dans la série de Volmar *et al.* qui regroupe 864 patients et qui concerne une série d'échantillons cytologiques bilio-pancréatiques, la sensibilité globale est de 52.6 %, la spécificité de 99,4 %, la valeur prédictive positive de 98.9 % et celle négative seulement de 67.1 % (Volmar KE *et al.* 2006;108(4):231-8).

Les performances de la cytologie en pathologie urologique, sont les suivantes :

| | Bas grade | Haut grade | Tous grades |
|---|---|---|---|
| Sensibilité | 26,8% | 77,5% | 61,9% |
| Spécificité | 93,1% | 93,1% | 93,1% |
| Valeur prédictive positive | 27,7% | 69,1% | 73,5% |
| Valeur prédictive négative | 92,8% | 95,5% | 88,8% |
| Précision du test | 87,2% | 90,5% | 85,5% |

D'après le Rapport de l'AFU : Tumeurs superficielles de vessie. Gattegno B., Chopin D. Prog Urol 1:867-875, 2001.

Compte-tenu de ces différents exemples de défaut de sensibilité pour le dépistage des tumeurs de bas grade de malignité, les inventeurs ont développé une méthode diagnostique innovante (Oncocell Test) combinant une coloration de Papanicolaou des échantillons cytologiques à leur analyse en fluorescence. Ceci permet de pouvoir détecter les lames contenant des cellules susceptibles de se répliquer ou se diviser (portant ainsi potentiellement des cellules tumorales). Une analyse complémentaire par imagerie en fond clair permet de poser un diagnostic définitif sur la nature des cellules observées.

Cette méthode permet de détecter les cellules capables de réplication. Ces cellules présentent une fluorescence localisée au niveau de leur membrane après coloration de Papanicolaou et excitation dans des gammes de longueur d'onde spécifiques. Les cellules ainsi fluorescentes incluent donc les cellules tumorales, mais également des cellules qui ne sont pas représentatives d'un état pathologique, telles que des cellules hématopoïétiques (polynucléaires, macrophages, leucocytes), des cellules des couches basales des différents épithéliums (cellules souches, progénitrices), des cellules capables de renouvellement (telles que des cellules épithéliales, des cellules glandulaires (notamment pancréatiques), ou des cellules épithéliales transformées par un virus (tel que le BK-virus ou le papillomavirus).

La coloration de Papanicolaou est une méthode bien connue en cytologie. Elle a été développée dès 1942 (voir p.ex. G. N. Papanicolaou: "A new procedure for staining vaginal smears", Science, vol. 95, no. 2469, avril 1942, p.438-439), afin de détecter les cancers cervico-utérins, et est réalisée de façon routinière dans les différents laboratoires de cytologie. Elle peut être réalisée de façon automatique dans des appareils existant sur le marché. Une variante de cette méthode comprend l'utilisation séquentielle de trois colorants :
- Hématoxyline : le produit de l'oxydation de hématoxyline, l'hématéine est un colorant anionique faible avec une faible affinité pour les tissus. Toutefois, l'hématéine devient un colorant qui présente une forte affinité pour les noyaux lorsqu'elle est combinée avec des éléments métalliques tel l'aluminium. On peut utiliser de l'hématoxyline de Harris ou de Mayer.
- L'Orange G : ce produit sert à la coloration des cytoplasmes en jaune, ou en orange en cas de présence de kératine.
- Le EA50 : il s'agit d'un mélange de plusieurs colorants : « light green », éosine Y et « Bismarck Brown ». Le « light green » est utilisé pour colorer le collagène. Il peut être remplacé par le « fast green », dont l'intensité diminue plus lentement. L'éosine Y sert à colorer le cytoplasme. Le « Brun Bismarck » n'est pas toujours présent dans IEA50, car il diminue la durée de conservation. Il colore les mucines acides.

Les échantillons sont rincés et déshydratés avec de l'alcool après chaque coloration.

On peut colorer les cellules avec l'hématoxyline de deux manières différentes : par la coloration progressive, la réaction est arrêtée une fois que l'on a atteint la coloration désirée. On peut également colorer par coloration régressive, dans laquelle on sature l'échantillon, puis on décolore par l'intermédiaire d'une solution diluée aqueuse ou alcoolisée d'acide chlorhydrique jusqu'à obtenir la coloration désirée.

Cette méthode est ainsi largement documentée dans la littérature, et bien connue de l'homme du métier.

Les inventeurs ont observé que les cellules capables de réplication, colorées par la coloration de Papanicolaou, présentent une fluorescence induite par excitation à une longueur d'onde inférieure à 520 nm, et notamment comprise entre 420 et 510 nm. Cette fluorescence est localisée au niveau de la membrane de ces cellules et est observée entre 520 et 650 nm. Karine Steenkeste ET AL: "Ex Vivo Fluorescence Imaging of Normal and Malignant Urothelial Cells to Enhance Early Diagnosis", Photochemistry and Photobiology, vol. 83, no. 5, septembre 2007, p. 1157-1166, décrivent la détection de cellules cancéreuses dans des échantillons de cytologie urinaire suite à une coloration de Papanicolaou utilisant l'hématoxyline, OG6 (Orange G-6) et EA50, suivie de l'observation de fluorescence périmembranaire.

L'invention se rapporte à une méthode
de détection de cellules capables de réplication dans un échantillon cytologique de cellules, ledit échantillon étant non gynécologique, comprenant la préparation et la coloration par la coloration de Papanicolaou, l'excitation dudit échantillon à une longueur d'onde inférieure à 520 nm, et l'observation de la fluorescence émise par ledit échantillon à une longueur d'onde supérieure à 520 nm, lesdites cellules capables de réplication présentant une accumulation de fluorescence localisée au niveau de leur enveloppe membranaire, caractérisé en ce que ladite coloration de Papanicolaou comprend les étapes successives suivantes, au cours desquelles ledit échantillon est mis en contact avec :
- Eau courante : 2 minutes
- Solution d'hématoxyline: 4 minutes
- Eau courante : 1 minute
- Solution comportant un mélange d'alcool 100% et d'HCI 0,2% : 30 secondes
- Eau courante : 10 minutes
- Eau ammoniaquée à 0,2% : 2 minutes
- Alcool à 70% : 30 secondes
- Alcool à 96% : 30 secondes
- Solution OG6 : 4 min
- Alcool à 96% : 30 secondes
- Alcool à 96% : 30 secondes
- Solution EA50 : 4 minutes
- Alcool à 96% : 30 secondes
- Alcool à 100% : 30 secondes
- Xylème : 30 secondes
- Xylène : 30 secondes,
puis montage sur monteuse de film sur lame en solution de xylène. La mise en oeuvre de cette méthode permet ainsi d'écarter les lames pour lesquelles on n'observe aucune fluorescence (et qui ne portent donc pas de cellules capables de réplication, donc pas de cellules tumorales). On peut alors préciser la nature des cellules fluorescentes sur les lames. Le cytologiste peut ainsi vérifier la nature des cellules fluorescentes par transmission classique, et identifier les autres signes de malignité éventuelle (taille des cellules, noyau, coloration
d'éléments internes (chromatine), condensation ou destruction de l'ADN nucléaire...), qui dépendent de l'origine de l'échantillon et de la pathologie considérée.

L'utilisation de la méthode selon l'invention permet ainsi au cytologiste de n'étudier que les lames susceptibles de porter des cellules tumorales. Par ailleurs, il lui suffit de n'observer, en transmission classique, que les zones contenant des cellules fluorescentes.

La mise en oeuvre de cette méthode permet ainsi de diminuer le temps passé à cribler les lames, sans risque de perte de qualité dans le diagnostic de cellules tumorales, en permettant, au contraire, de détecter plus précocement ces cellules tumorales.

Dans le cadre de la présente invention, un « échantillon cytologique de cellules » représente un échantillon de cellules qui a été déposé sur lames cytologiques selon les méthodes connues dans l'art, et qui seront rappelées ci-après.

Il convient de noter que l'observation sous lumière fluorescente de lames colorées par la coloration de Papanicolaou a déjà été décrite dans l'art antérieur. Ainsi, Küpper et al. (Cytopathology, 1995, 6(5), 331-338) rapporte le diagnostic de tuberculose due à *Mycobacterium kansasi* sur des échantillons cytologiques.

Ghali et al. (Hum Pathol. 1984, 5(10), 907-9) rapportent que des *Pneumocystis carinii* présentent une fluorescence lorsque l'on soumet des échantillons colorés par Papanicolaou à une lumière infrarouge.

Une fluorescence de microorganismes dans des échantillons colorés par la coloration de Papanicolaou a également été rapportée pour des *Aspergillus* (Hettlich et al, Cytopathology. 1998, 9(6), 381-8).

On peut également détecter des cristaux de Charcot-Leiden en soumettant des échantillons colorés par la coloration de Papanicolaou à une fluorescence (Küpper et al, Cytopathology. 1994, 5(5), 262-9).

La mise en oeuvre du procédé selon l'invention contient plusieurs étapes, entre le prélèvement effectué sur le patient, et l'observation finale des lames sous fluorescence (suivie éventuellement de la caractérisation des cellules fluorescentes par observation par microscopie optique sous lumière claire).

### Préparation des lames cytologiques

La première étape est la fixation des cellules après prélèvement sur le patient, puis leur dépôt sur lame cytologique.

L'objectif de l'étape de fixation est de maintenir les cellules de l'échantillon biologique dans un état qui soit le plus proche de l'état physiologique naturel. En particulier, on souhaite préserver au maximum l'intégrité de la membrane et de la matrice extracellulaire. Il convient également de préserver les détails morphologiques de la cellule, d'éviter une activation des enzymes extracellulaires et des protéines de coagulation.

On effectue ainsi préférentiellement une fixation chimique, en plongeant le prélèvement dans un liquide contenant un agent fixateur.

De nombreux agents fixateurs présentant ces propriétés sont connus dans l'art. Ainsi, on peut noter les agents à base de formaldéhyde qui sont particulièrement adaptés pour la mise en oeuvre de l'étape de fixation des cellules sur la lame cytologique. Ces agents fixateurs à base de formaldéhyde peuvent être tamponnés pour présenter un pH neutre. Ils peuvent être aqueux ou à base d'alcool.

Des agents à base d'alcool (méthanol) peuvent également être employés.

On peut aussi travailler sur des échantillons liquides dans lesquels les cellules sont conservées dans un milieu tel que le Cytolyt® (Hologic Inc, Bedford, MA, USA), et de resuspendre les cellules rapidement dans un milieu tel que le PreservCyt® (Hologic Inc, Bedford, MA, USA). La solution CytoLyt® est une solution de conservation tamponnée à base de méthanol conçue pour lyser les hématies, éviter la précipitation des protéines, dissoudre le mucus, et préserver la morphologie des échantillons cytologiques normaux. Elle constitue un milieu de transport et est utilisée lors de la préparation d'un échantillon avant son traitement. Elle n'a pas pour objet la destruction microbienne totale.

Ainsi que mentionné plus haut, cette étape de fixation chimique des cellules permet de maintenir au maximum les cellules du prélèvement dans un état le plus proche de l'état physiologique, et optimise ainsi la mise en oeuvre de la méthode selon l'invention.

Il est donc préféré que cette étape de fixation de l'échantillon cellulaire soit réalisée le plus rapidement possible (et de préférence immédiatement) à la suite du prélèvement biologique. En tout état de cause, on préfère lorsque ledit échantillon a été fixé dans les 3 heures, de préférence dans l'heure, de façon plus préférée 20 minutes ou moins, de façon la plus préférée dans les 5 minutes suivant le prélèvement biologique.

Une fois fixé chimiquement, l'échantillon cellulaire est déposé et étalé sur lame. L'échantillon est alors « séché » afin d'assurer une bonne adhérence sur la lame avant coloration. Ce séchage peut être réalisé à l'air, dans une étuve, à l'alcool (tel que isopropane2ol, éthanol), ou en utilisant un spray fixateur. Dans ce dernier cas, il est commun que le fixateur contienne des copolymères (tels que le poly-éthylène-glycol) qui laisse un mince film sur l'échantillon. Il est donc préférable de rincer la lame pour éliminer ce film protecteur et garantir une coloration optimale des cellules. La lame est rincée à l'eau si les copolymères du spray sont hydrosolubles, ou avec de l'alcool.

On préfère réaliser cette étape de dépôt / étalement sur lame rapidement après la fixation de l'échantillon.

Ainsi, on préfère lorsque ledit échantillon a été fixé sur la lame dans les 72 heures suivant le prélèvement biologique, de préférence dans les 48 heures, suivant le prélèvement, de manière préférée dans les 24 heures suivant le prélèvement, de manière encore plus préférée dans les 12 heures suivant le prélèvement biologique.

Toutefois, certains fixateurs liquides peuvent permettre de conserver les échantillons pendant un temps relativement long, sans qu'ils ne se dégradent.

Il est entendu que l'homme du métier peut aisément trouver dans l'art antérieur différents protocoles de préparations de lames cytologiques pour des échantillons d'origine ORL, urinaire, gynécologique, thyroïdienne, mammaire, hématologique, provenant des séreuses (péritoine, plèvre, péricarde, méninges), bronchique ou autres. Toutefois, même si les modalités de fixation peuvent varier en fonction de l'organe d'origine du prélèvement, la fixation des cellules dans un fixateur liquide rapidement après prélèvement permet de favoriser les performances de la méthode selon l'invention, ainsi qu'indiqué plus haut. Alternativement le dépôt sur lame avec fixation chimique sur la lame permettant de maintenir les cellules de l'échantillon biologique dans un état qui soit le plus proche de l'état physiologique naturel peut être envisagé.

### Coloration des lames cytologiques

Après dépôt des échantillons biologiques sur lames cytologiques, celles-ci sont colorées par la méthode de Papanicolaou, en utilisant séquentiellement les trois colorants rappelés plus haut.

Dans un mode de réalisation préféré, on colore les échantillons cytologiques moins de 24 heures (quelques minutes à quelques heures (6 à 12 heures)) après dépôt sur lame. Il est préférable d'effectuer la coloration de Papanicolaou dans les 5 jours suivant la fixation sur la lame cytologique, et de préférence dans les 48 heures suivant la fixation sur la lame cytologique.

Ainsi que vu plus haut, il existe une grande variété dans l'application des différents colorants selon les protocoles de coloration de Papanicolaou.

Toutefois, les inventeurs ont mis en évidence le fait que la fluorescence observée au niveau membranaire est due à la localisation de l'EA50 à ce niveau. Il est donc préférable de ne pas saturer la lame avec ce colorant. Ainsi, dans le mode de réalisation de l'invention, on colore ledit échantillon avec IEA50 pendant 4 minutes.

On rince l'échantillon, après coloration avec IEA50, une première fois avec de l'alcool à 96% pendant 30 secondes, puis une seconde fois avec de l'alcool à 100% pendant 30 secondes. On applique ensuite le xylène permettant de finir la coloration. Ainsi, dans ce mode de réalisation, on limite les étapes de rinçage à l'alcool après coloration avec l'EA50.

D'une façon générale, il est préférable de mettre en oeuvre la méthode de coloration de telle sorte que les rinçages après chaque étape de coloration soient efficaces. Les rinçages aqueux se font sous flux continu. Les rinçages alcooliques sont courts, typiquement 30 secondes.

### Observation des lames cytologiques

Ainsi que vu plus haut, les inventeurs ont observé que les cellules capables de réplication présentes dans un échantillon cytologique coloré à l'aide d'une coloration de Papanicolaou, émettent un signal de fluorescence cellulaire lorsque l'on les soumet à une longueur d'onde excitatrice. Ce signal de fluorescence est induit par une excitation en lumière visible ou proche UV (longueur d'onde inférieure à 520 nm), l'émission de fluorescence étant collectée au-delà de 520 nm.

De façon préférentielle, la longueur d'onde d'excitation est comprise entre 420 et 510 nm, de façon plus préférée entre 450 et 490 nm.

Les inventeurs ont pu déterminer que l'accumulation de fluorescence observée au niveau de l'enveloppe membranaire des cellules en division émet à une longueur d'onde centrée sur 550 nm. Ainsi, même s'il est possible de détecter cette fluorescence dans une plage plus large (520 à 620 nm), on préfère travailler à cette longueur d'onde ou détecter la fluorescence émise dans la plage comprise entre 540 et 560 nm.

### Mise en oeuvre de la méthode

La mise en oeuvre de cette méthode permet ainsi de trier parmi une série de lames de cytologie celles pour lesquelles on n'observe aucune fluorescence perimembranaire (et qui ne contiennent donc pas de cellules tumorales), et celles pour lesquelles il existe des cellules qui émettent de la fluorescence au niveau de la membrane cytoplasmique. Ces cellules nécessiteront un contrôle de la nature tumorale ou non de ces cellules par un examen en lumière à fond clair.

Le cytopathologiste peut identifier en transmission classique, les signes de malignité éventuels (taille des noyaux, hyperchromatisme, taille des nucléoles, augmentation du rapport nucléo-cytoplasmique....), qui dépendent de l'origine de l'échantillon et de la pathologie considérée.

Compte tenu de la forte Valeur Prédictive Négative de la méthode selon l'invention, sa mise en oeuvre permet ainsi au cytopathologiste, de n'étudier que les lames susceptibles de porter des cellules tumorales.

L'un des avantages majeurs de la méthode selon l'invention est qu'elle utilise les lames traditionnellement observées par les cytopathologistes, mais fournit des informations complémentaires (fluorescence), sans ajouter de contrainte particulière de préparation.

La mise en oeuvre de cette méthode permet ainsi de diminuer le temps passé à cribler les lames, sans risque de perte de qualité dans le diagnostic et d'établir, au contraire, un diagnostic plus précoce des cellules tumorales.

### Domaine d'application de la méthode

La méthode ici décrite a notamment été validée sur des échantillons de cytologies urinaires, cytologies gynécologiques et endométriales (frottis cervico-utérins), cytologies pancréatiques et cytologies mammaires.

Toutefois, cette méthode est également applicable pour d'autres cytologies telles que cytologies : ponctions d'organes superficiels (thyroïde, sein, ganglion lymphatique), ponction d'organes profonds (médiastin, ganglion lymphatique, moelle osseuse, poumons, foie, pancréas, voies biliaires, paroi digestive, surrénale, rein, toute masse profonde abdominale ou rétropéritonéale, glandes salivaires), recueil de cellules dans des liquides physiologiques (séreuses : liquide pleural, liquide d'ascite ; urines ; bile ; liquide articulaire ; liquide céphalo-rachidien ; hématologie), recueil de cellules par brossage (bronchique, urinaire et biliaire) et recueil de cellules après injection de sérum physiologique : cytologie respiratoire telle que le lavage bronchiolo-alvéolaire, frottis anaux, sphère ORL, cytologie de la prostate (massage prostatique et recueil de la première urine après massage).

### Automatisation de la méthode

Ainsi que vu plus haut, il est intéressant de pouvoir automatiser la première lecture des lames cytologiques, afin que le cytologiste n'observe que les lames dans lesquelles une fluorescence a été observée.

Cette automatisation des différentes étapes de la méthode devrait ainsi permettre un gain de temps pour le cytopathologiste, ainsi qu'une excellente standardisation.

Il est ainsi envisagé que l'analyse des lames soit réalisée après numérisation de celles-ci à l'aide d'un scanner, ou capture d'image par un appareil photographique.

Ainsi les lames sont analysées en fluorescence à différents grandissements, pour mettre en évidence les signatures spécifiques des cellules capables de réplication. Les lames scannées et analysées permettent de présenter une lame virtuelle annotée et classée au pathologiste pour un gain de temps et de sensibilité majeurs. Ce scanner couplé à une caméra est réglé de telle manière qu'il puisse déterminer l'émission de fluorescence dans les longueurs d'ondes précisées ci-dessus, et centrées autour de 550 mn sous une onde d'excitation comprise entre 420 et 510 nm (de préférence entre 450 et 490 nm).

On peut également capturer les images des mêmes lames sous lumière claire, le cytopathologiste observant alors simultanément l'image de la lame sous fluorescence et sous lumière claire, et pouvant établir le diagnostic de malignité.

Bien entendu, à tout moment, le cytopathologiste peut revenir aux lames originelles pour les observer directement en lumière à fond clair et contrôler les amas de cellules douteuses.

Des logiciels d'analyse d'image et de reconnaissance de zones d'intérêt sur la lame permettent au cytopathologiste de bénéficier d'une aide à la lecture par une sélection des champs à étudier.

Ainsi, on peut utiliser pour la mise en oeuvre de la méthode selon l'invention, un dispositif de lecture d'une lame cytologique colorée par une coloration de Papanicolaou, qui contient des moyens d'émission d'une longueur d'onde d'excitation sur ladite lame, des moyens de lecture de la fluorescence émise par ladite lame.
Les moyens d'émission d'une longueur d'onde d'excitation sur la lame cytologique comprennent une source de radiation émettant dans une gamme de longueur d'ondes allant de 420 à 510 nm, de préférence de 450 à 490 nm. Il peut s'agir d'un laser.
Les moyens de lecture de la fluorescence émise par ladite lame (plus précisément par les cellules présentes sur la lame) comprennent un capteur permettant de mesurer la radiation émise par la lame après excitation. Ce capteur peut également déterminer la zone (ou les zones) de la lame dans laquelle l'émission de fluorescence est réalisée. Ce capteur est réglé de telle manière qu'il puisse déterminer l'émission de fluorescence dans les longueurs d'ondes précisées ci-dessus, et centrées autour de 550 nm.
De préférence, ce dispositif comprend également des moyens de capture d'images de ladite lame (ou de plusieurs zones de ladite lame).
Ces moyens de capture peuvent être un appareil photo qui pourra capturer l'image de la lame (ou de cellules de la lame) émettant une fluorescence après excitation. Il est également préféré lorsque le dispositif permet la capture de
l'image de la même zone sous illumination directe (c'est-à-dire sans excitation des échantillons).

De manière préférée, ces images capturées sont stockées dans une mémoire (présente sur l'appareil ou déportée), de telle façon que le cytologiste puisse y accéder par un moyen informatique. Ces images peuvent également être imprimées.

Ainsi, le cytologiste pourra vérifier les échantillons à la fois en comparant les images présentant les cellules fluorescentes et les images présentant la structure des cellules, afin de déterminer la présence de cellules tumorales et définir un diagnostic. Le dispositif peut comprendre des moyens associés au capteur de radiation, permettant d'analyser la lame illuminée pour déterminer les zones susceptibles de présenter des cellules tumorales, notamment en fonction de la fluorescence émise (intensité, forme de l'élément fluorescent), et qui doivent être étudiées de façon plus précise. Ceci permet ainsi d'identifier les cellules d'intérêt (présentant une fluorescence périphérique) sur la lame, d'en prendre une photo, de les éclairer et de les photographier sous lumière non-fluorescente.

Certaines demandes de brevets (notamment WO 96/041303 ou WO 96/009593) décrivent des moyens utilisables pour la détermination de zones d'intérêt sur une lame, ainsi que des moyens permettant de classifier les échantillons présents sur la lame cytologique en fonction notamment de la fluorescence émise, ainsi que décrit. Ainsi, on sait que les cellules d'origine sanguine présentent un diamètre inférieur aux cellules tumorales dans un certain nombre d'échantillons. On peut donc préciser un niveau de diamètre en dessous duquel les cellules émettant la fluorescence ne sont pas considérées comme tumorales et non prises en compte. Le dispositif peut comprendre en outre des moyens permettant de colorer les lames cytologiques par la méthode de Papanicolaou. On peut effectuer cette coloration en plongeant les lames cytologiques dans des bacs, ou en utilisant des sprays, ainsi que décrit dans WO 92/019952. L'automatisation de la coloration de lames par la méthode de Papanicolaou ne présente pas de difficultés techniques. Un procédé automatisé de détection de cellules ayant un potentiel de réplication sur un échantillon cytologique de cellules, est caractérisé en ce qu'il comprend les étapes consistant à :
a) Soumettre la lame comprenant ledit échantillon à une longueur d'onde comprise entre 420 et 510 nm (de préférence entre 450 et 490 nm)
b) Identifier les zones éventuelles de la lame dans lesquelles une fluorescence est émise autour de 550 nm
c) Capturer une image de ces zones sous fluorescence
d) Capturer une image de ces zones en microscopie par transmission classique, sous lumière visible
e) Stocker les images obtenues en c) et d), de telle sorte que les images soient associées à la lame observée

Les étapes a) à e) peuvent être répétées avec une nouvelle lame.

### Description des Figures

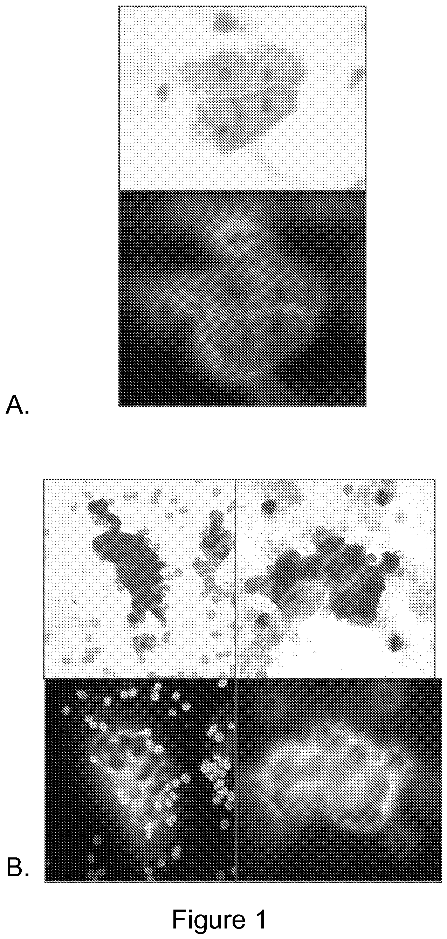

Figure 1 : la figure 1.A montre la fluorescence observée pour des cellules urothéliales saines. La fluorescence est diffuse dans le cytoplasme de ces cellules.
La figure 1.B montre l'accumulation de fluorescence sur l'enveloppe membranaire de cellules cancéreuses (carcinome urothélial).

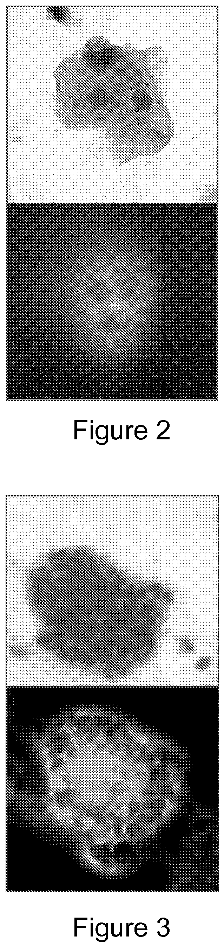

Figure 2: cellules malpighiennes (saines) en microscopie classique et sous fluorescence. La fluorescence est diffuse dans le cytoplasme de ces cellules.
Figure 3 : polynucléaires neutrophiles observés en microscopie classique et sous fluorescence. Ces cellules, qui ont la capacité de se répliquer, présentent une fluorescence accumulée sur l'enveloppe membranaire.

### Exemples de préparation des échantillons cellulaires en pathologie non gynécologique

### Exemple 1 Préparation de lames à partir d'échantillons d'organes profonds

### a) Pour les cellules reçues en suspension dans le CytoLyt®.

L'échantillon est concentré par centrifugation (par exemple 600G pendant 10 minutes), et le surnageant est éliminé.

L'échantillon est ensuite rincé dans une solution du type PreservCyt® (Hologic Inc). Le temps de contact avec la solution PreservCyt est au moins de 15 mn. Le PreservCyt® est une solution tamponnée de méthanol qui permet la conservation des cellules entre 15° C et 30° C dans les 6 semaines.

On peut ensuite fixer les cellules sur lame en utilisant l'automate ThinPrep (Hologic).

Le flacon d'échantillon ThinPrep est placé dans un processeur ThinPrep 2000 où une dispersion douce, désagrège le sang, le mucus et les débris impropres au diagnostic, et mélange les cellules de l'échantillon. Les cellules sont ensuite recueillies sur un filtre conçu à cet effet et spécifique aux prélèvements non gynécologiques. Le processeur contrôle le débit à travers le filtre de façon à éviter qu'elles ne soient pas trop abondantes ni trop rares.

On renverse alors le filtre sur la lame. Une attraction naturelle et une pression d'air légèrement positive permettent l'adhérence des cellules sur la lame. Il en résulte une répartition égale des cellules dans une zone circulaire définie.

Cette lame est ainsi automatiquement déposée dans une solution de fixation contenant de l'alcool à 95° pendant au moins 10 minutes avant toute coloration. Une fine couche de cellules est alors transférée sur une lame de verre dans un cercle de 20 mm de diamètre.

On peut ensuite effectuer la coloration.

### Exemple 2 : Cytopathologie urinaire, brossages de l'appareil urinaire

Les échantillons arrivent fixés dans du formol : 30 ml d'urines fraîches pour 4 ml de formol à 10% dans un tube conique type 50 ml.

La technique est réalisée comme indiquée ci-dessous :
- la lame identifiée est introduite dans le Shandon Mégafunnel® (ThermoFisher)
- 2 ml d'urines sont déposées dans le réservoir du Mégafunnel® ; le reste des urines est conservé une semaine maximum au réfrigérateur.
- le Mégafunnel® est déposé dans le tambour de la cytocentrifugeuse Thermo Electron® à une vitesse de 4 à 400 à 800 tours/minute pendant 10 minutes (programme 2).
- A l'arrêt de la centrifugation, on récupère la lame et on la fixe immédiatement avec le spray fixant ou alcool à 70°C pendant % d'heure.

On peut ensuite effectuer la coloration.

### Exemple 3 : Cytopathologie biliaire

### a) brossage biliaire

La brosse arrive dans un tube conique contenant du CytoLyt®
- Vortexer la brosse
- Retirer la brosse et la mettre dans un autre tube stérile dans du sérum physiologique.
- Mettre à centrifuger 1500 tours/mn pendant 10 mn dans la centrifugeuse JOUAN le tube à fond conique type FALCON
- Jeter le surnageant dans le récipient déchet liquide
- Ajouter un peu de solution PreservCyt® sur le culot
- Bien agiter et transvaser le culot en totalité dans un pot de PreservCyt® de 30 ml.
- Laisser en contact au minimum 15 mn mais peut attendre plusieurs jours à température ambiante
- Préparer une lame Thinprep (voir exemple 1)

### b) Bile

### Réception des liquides à l'état frais

On prépare les lames de la même façon qu'à l'exemple 2 (cytofunnel) et puis on peut les colorer.

### Exemple 4 : coloration de Papanicolaou

- Eau courante : 2 min
- Solution d'hématoxyline: 4 min
- Eau courante : 1 min
- Solution comportant un mélange d'alcool 100% et d'HCI 0.2% : 30 sec
- Eau courante : 10 min
- Eau ammoniaquée à 0.2% : 2 min
- Alcool 70% : 30 sec
- Alcool 96% : 30 sec
- Solution OG6 : 4 min
- Alcool 96% : 30 sec
- Alcool 96% : 30 sec
- Solution EA50 : 4 min
- Alcool 96% : 30 sec
- Alcool 100% : 30 sec
- Xylène : 30 sec
- Xylène : 30 sec
- Montage sur monteuse de film sur lame en solution xylène.

| Composants | Référence | Fournisseur | Conditionnement |
|---|---|---|---|
| Papanicolaou solution 3b Solution polychrome EA50 | HX804463 | Merck KGaA | Bouteille de 2,5 l |
| Papanicolaou solution 2a Orangé G en solution (OG6) | HX815072 | Merck KGaA | Bouteille de 2,5 l |
| Hémalun en solution selon Mayer | HX811422 | Merck KGaA | Bouteille de 2,5 l |

Merck KGaA, 64271 Darmstadt, Germany.

### Exemple 5 : lecture des échantillons

La lecture se fait sur un microscope optique à épifluorescence (excitation longueurs d'onde <520 nm ; émission de fluorescence longueurs d'onde >520 nm). Un tel microscope équipe tous les laboratoires d'Anatomie et de Cytologie Pathologiques pour le travail de routine.

L'interprétation des cytologies par le pathologiste est facilitée : le « screening » des cellules tumorales se fait en première intention sous épifluorescence, les caractéristiques des cellules en division étant une accumulation de fluorescence dans l'enveloppe membranaire. Les cellules quiescentes (qui ne se divisent pas) présentent une fluorescence diffuse intra-cytoplasmique. Il convient de noter que les cellules en division présentent également cette fluorescence cytoplasmique, qui est toutefois peu visible du fait de l'importance de la fluorescence liée à la membrane cellulaire.

Lorsque des cellules cancéreuses potentielles sont détectées, on peut effectuer une confirmation en microscopie en fond clair

Il convient de noter qu'une diminution du signal fluorescent (fading) peut être observée au fur et à mesure du temps d'observation et au fil du temps. Une conservation des lames à l'abri de la lumière est souhaitable.

Cette méthode permet ainsi de repérer rapidement des cellules cancéreuses dans un échantillon, lorsqu'elles sont présentes en très faible quantité.

### Exemple 6 : résultats biologiques

Une étude a été réalisée sur des cytologies faites chez des patients ayant un carcinome urothélial de vessie connu, chez des témoins non atteints de cancer ou sur des cytologies classées douteuses.

42 patients avec carcinomes urothéliaux ont été inclus dont : 22 grade 1 (G1), 5 grade 2 (G2) et 15 grade 3 (G3).

101 patients indemnes de pathologie tumorale ont aussi été analysés selon le même protocole, ainsi que 26 patients ayant une cytologie douteuse.

Résultats : La synthèse des résultats est décrite dans les tableaux suivants.

| **Cancers urothéliaux (n=42)** | **Cytologie -** | **Cytologie +** | **Cytologie douteuse** |
|---|---|---|---|
| **Fluorescence -** | 0 | 0 | 0 |
| **Fluorescence +** | 4 | 24 | 14 |
| | G1 : 2 | G1 : 10 | G1 : 10 |
| **Répartition selon les** | G2 : 2 | G2 : 0 | G2 : 3 |
| | G3 : 0 | G3 : 14 | G3 : 1 |

| | | | |
|---|---|---|---|
| Sensibilité Cytologie : 24/42 = 57%, (pour les G1/G2 = 10/27 (37%) G3 = 14/15 (93%)) Sensibilité Fluorescence : 42/42 (100%) | | | |

| **Patients sans cancers (n = 101)** | **Cytologie -** | **Cytologie +** | **Cytologie douteuse** |
|---|---|---|---|
| **Fluorescence -** | 59 | 1 | 36 |
| **Fluorescence +** | 1 | 2 | 2 |

| | | | |
|---|---|---|---|
| Sensibilité : Cytologie 60/101 = 59% Sensibilité : Fluorescence : 96/101= 95% | | | |

Par ailleurs, 26 cytologies suspectes ont été réévaluées avec la fluorescence : 17 tests en fluorescence positifs (17 carcinomes découverts), 3 tests en fluorescence négatifs (3 cystites), 6 tests en fluorescence positifs (5 cystites et 1 lithiase).

La méthode selon l'invention permet donc bien d'améliorer l'étude cytologique d'échantillons biologiques urinaires et d'augmenter la sensibilité et la Valeur Prédictive Négative.

## Revendications

1. Méthode de détection de cellules capables de réplication dans un échantillon cytologique de cellules, ledit échantillon étant non gynécologique, comprenant la préparation et la coloration par la coloration de Papanicolaou, l'excitation dudit échantillon à une longueur d'onde inférieure à 520 nm, et l'observation de la fluorescence émise par ledit échantillon à une longueur d'onde supérieure à 520 nm, lesdites cellules capables de réplication présentant une accumulation de fluorescence localisée au niveau de leur enveloppe membranaire, **caractérisé en ce que** ladite coloration de Papanicolaou comprend les étapes successives suivantes, au cours desquelles ledit échantillon est mis en contact avec :
- Eau courante : 2 minutes
- Solution d'hématoxyline: 4 minutes
- Eau courante : 1 minute
- Solution comportant un mélange d'alcool 100% et d'HCI 0,2% : 30 secondes
- Eau courante : 10 minutes
- Eau ammoniaquée à 0,2% : 2 minutes
- Alcool à 70% : 30 secondes
- Alcool à 96% : 30 secondes
- Solution OG6 : 4 min
- Alcool à 96% : 30 secondes
- Alcool à 96% : 30 secondes
- Solution EA50 : 4 minutes
- Alcool à 96% : 30 secondes
- Alcool à 100% : 30 secondes
- Xylène : 30 secondes
- Xylène : 30 secondes,
puis montage sur monteuse de film sur lame en solution de xylène.

2. Méthode selon la revendication 1, **caractérisée en ce que** la longueur d'onde d'excitation est comprise entre 450 et 490 nm.

3. Méthode selon l'une des revendications 1 ou 2, **caractérisée en ce que** l'on observe la fluorescence émise à des longueurs d'onde entre 540 et 560 nm.

4. Méthode selon l'une des revendications 1 à 3, **caractérisée en ce que** ledit échantillon a été fixé de telle sorte que l'intégrité de la membrane et de la matrice extracellulaire des cellules de l'échantillon a été maintenue.

5. Méthode selon l'une des revendications 1 à 4, **caractérisée en ce que** la fixation des échantillons est effectuée avec un fixateur contenant du formaldéhyde ou du méthanol.

6. Méthode selon l'une des revendications 1 à 5, **caractérisée en ce que** ledit échantillon a été fixé dans les 3 heures suivant le prélèvement biologique.

7. Méthode selon la revendication 6, **caractérisée en ce que** ledit échantillon a été fixé 20 minutes ou moins après le prélèvement biologique.

8. Méthode selon l'une des revendications 1 à 7, **caractérisé en ce que** ledit échantillon est choisi parmi un échantillon de cytologie urinaire, un échantillon de cytologie thyroïdienne, un échantillon de cytologie des glandes salivaires, un échantillon de cytologie respiratoire, un échantillon de cytologie des séreuses, un échantillon de cytologie bilio-pancréatique, un échantillon de cytologie hématologique, un échantillon de cytologie du système nerveux, un échantillon de cytologie prostatique.

## Patentansprüche

1. Verfahren zum Nachweis replikationskompetenter Zellen in einer zytologischen Zellprobe, wobei die Probe nicht gynäkologisch ist, umfassend die Zubereitung und die Färbung durch die Papanicolaou-Färbung, die Anregung der Probe bei einer Wellenlänge unter 520 nm und die Beobachtung der von der Probe bei einer Wellenlänge über 520 nm emittierten Fluoreszenz, wobei die replikationskompetenten Zellen eine Akkumulation der Fluoreszenz aufweisen, die an ihrer Membranhülle lokalisiert ist, **dadurch gekennzeichnet, dass** die Papanicolaou-Färbung die folgenden aufeinanderfolgenden Schritte umfasst, in denen die Probe mit Folgendem in Kontakt gebracht wird:
- fließendem Wasser : 2 Minuten
- Hämatoxylin-Lösung: 4 Minuten
- fließendem Wasser : 1 Minute
- Lösung mit einem Gemisch von 100% Alkohol und 0,2% HCl: 30 Sekunden
- fließendem Wasser : 10 Minuten
- 0,2% Ammoniak-Wasser: 2 Minuten
- 70% Alkohol: 30 Sekunden
- 96% Alkohol: 30 Sekunden
- OG6-Lösung : 4 min
- 96% Alkohol: 30 Sekunden
- 96% Alkohol: 30 Sekunden
- EA50-Lösung: 4 Minuten
- 96% Alkohol: 30 Sekunden
- 100% Alkohol: 30 Sekunden
- Xylol: 30 Sekunden
- Xylol: 30 Sekunden,
und anschließendes Montieren an einer Filmmontagevorrichtung auf einen Objektträger in Xylol-Lösung.

2. Verfahren nach Anspruch 1, **dadurch gekennzeichnet, dass** die Anregungswellenlänge zwischen 450 und 490 nm beträgt.

3. Verfahren nach einem der Ansprüche 1 oder 2, **dadurch gekennzeichnet, dass** man die bei Wellenlängen zwischen 540 und 560 nm emittierte Fluoreszenz beobachtet.

4. Verfahren nach einem der Ansprüche 1 bis 3, **dadurch gekennzeichnet, dass** die Probe derart fixiert wurde, dass die Unversehrtheit der Membran und der extrazellulären Matrix der Zellen der Probe aufrechterhalten wird.

5. Verfahren nach einem der Ansprüche 1 bis 4, **dadurch gekennzeichnet, dass** die Fixierung der Proben mit einem Fixiermittel durchgeführt wird, das Formaldehyd oder Methanol enthält.

6. Verfahren nach einem der Ansprüche 1 bis 5, **dadurch gekennzeichnet, dass** die Probe in den 3 Stunden nach der Entnahme der biologischen Probe fixiert wird.

7. Verfahren nach Anspruch 6, **dadurch gekennzeichnet, dass** die Probe 20 Minuten oder weniger nach der Entnahme der biologischen Probe fixiert wurde.

8. Verfahren nach einem der Ansprüche 1 bis 7, **dadurch gekennzeichnet, dass** die Probe aus einer Urinzytologieprobe, einer Schilddrüsenzytologieprobe, einer Speicheldrüsenzytologieprobe, einer Atemwegszytologieprobe, einer Serosazytologieprobe, einer Gallen-Pankreas-Zytologieprobe, einer hämatologischen Zytologieprobe, einer Nervensystemzytologieprobe, einer Prostatazytologieprobe ausgewählt ist.

## Claims

1. Method of detecting cells capable of replication in a cytological sample of cells, said sample being non-gynaecological, comprising preparation and staining by Papanicolaou staining, excitation of said sample at a wavelength below 520 nm, and observation of the fluorescence emitted by said sample at a wavelength above 520 nm, said cells capable of replication exhibiting an accumulation of fluorescence localized at their membrane envelope, **characterized in that** said Papanicolaou staining comprises the following successive steps; during which said sample is brought into contact with:
• Running water: 2 min
• Hematoxylin solution: 4 min
• Running water: 1 min
• Solution comprising a mixture of 100% alcohol and of 0.2% HCl: 30 sec
• Running water: 10 min
• Aqueous ammonia solution at 0.2%: 2 min
• 70% alcohol: 30 sec
• 96% alcohol: 30 sec
• OG6 solution: 4 min
• 96% alcohol: 30 sec
• 96% alcohol: 30 sec
• EA50 solution: 4 min
• 96% alcohol: 30 sec
• 100% alcohol: 30 sec
• Xylene: 30 sec
• Xylene: 30 sec
followed by mounting on a film on slide mounter in xylene solution.

2. Method according to Claim 1, **characterized in that** the excitation wavelength is between 450 and 490 nm.

3. Method according to either of Claims 1 and 2, **characterized in that** the fluorescence emitted at wavelengths of between 540 and 560 nm is observed.

4. Method according to one of Claims 1 to 3, **characterized in that** said sample was fixed in such a way that the integrity of the membrane and of the extracellular matrix of the cells of the sample was maintained.

5. Method according to one of Claims 1 to 4, **characterized in that** the fixing of the samples is carried out with a fixative containing formaldehyde or methanol.

6. Method according to one of Claims 1 to 5, **characterized in that** said sample was fixed within 3 hours following the taking of the biological sample.

7. Method according to Claim 6, **characterized in that** said sample was fixed 20 minutes or less after the taking of the biological sample.

8. Method according to one of Claims 1 to 7, **characterized in that** said sample is chosen from a urinary cytology sample, a thyroid cytology sample, a salivary gland cytology sample, a respiratory cytology sample, a serous membrane cytology sample, a bilio-pancreatic cytology sample, a hematological cytology sample, a nervous system cytology sample and a prostate cytology sample.
